# EUROPEAN PATENT APPLICATION

(11) **EP 3 795 236 A1**
(43) Date of publication of application: **24.03.2021**
(21) Application number: 19198450.9
(22) Date of filing: 19.09.2019
(51) Int. Cl.: B01D 53/14, B01D 53/62, B01D 53/78, C07C 51/41, C07C 51/44, C07C 401/00, B01D 53/50

(54) **FORMIC ACID FORMATION FROM A CARBON DIOXIDE SOURCE**

(71) Applicant: Nederlandse Organisatie voor toegepast- natuurwetenschappelijk Onderzoek TNO, 2595 DA 's-Gravenhage (NL)
(72) Inventor: The designation of the inventor has not yet been filed
(74) Representative: Nederlandsch Octrooibureau

(57) **Abstract**

The present invention concerns a process for producing formic acid, comprising (a) a carbon capture step wherein a source of carbon dioxide is contacted with an amine solution in a closed-top scrubber, to obtain an ammonium bicarbonate solution; (b) inducing crystallization in the ammonium bicarbonate solution to obtain a concentrated ammonium bicarbonate solution; (c) subjecting the concentrated ammonium bicarbonate solution to a hydrogenation step to obtain an ammonium formate; and (d) heating the ammonium formate to a temperature in the range of 50 - 150 °C, to obtain a gaseous product containing the amine and a liquid product stream containing formic acid. Herein, the source of carbon dioxide has a carbon dioxide content of at least 95 vol% and the amine used in step (a) and reformed in step (d) has a partial vapour pressure above a 40 wt% solution of the amine in water at 20 °C of at least 40 kPa. The present invention further concerns a system for performing the process according to the invention.

## Description

### Field of the invention

The present invention relates to a reactor and a process for the formation of formic acid from carbon dioxide.

### Background art

During the last decades, consensus is growing that carbon dioxide emissions are increasing to alarming levels. Capturing of carbon dioxide constitute an important technology towards the minimization of such emissions. The overall success of carbon dioxide capture for a large part resides in the possibility of effectively transforming the captured carbon dioxide into valuable end-products.

Formic acid and formaldehyde are important industrial chemicals with many applications as end-product or precursor to more complex molecules. Millar et al. in Ind. Eng. Chem. Res. 2017, 56(33), 9247-9265 discloses a process for the industrial production of formaldehyde. Conventional process for the formation of formic acid from carbon dioxide is reviewed by Rumayor et al. in Appl. Sci. 2008, 8(6), 914. The inventors have developed an improved process wherein captured carbon dioxide is efficiently converted into formic acid or formaldehyde.

### Summary of the invention

The invention concerns a highly efficient and durable process for the production of formic acid from carbon dioxide. The inventors have for the first time combined these steps in an efficient and durable process for the production of formic acid. In the process according to the invention, carbon dioxide is first captured in an amine solution with the help of an amine solution, hereby converting the carbon dioxide to ammonium bicarbonate salts. The ammonium salts precipitate out and form a slurry together with the solvent, and can then be removed from the slurry by a crystallization step. By hydrogenating the ammonium salts, ammonium formate salts are formed. To produce formic acid the mixture is heated in a stripper column and the ammonium formate splits into formic acid and the amine. Amine is discharged from the top of the stripper column and formic acid and possibly other remaining liquid components exit from the bottom of the stripper column.

The invention may be defined according to the following list of preferred embodiments:
1. A process for producing formic acid, comprising:
   (a) a carbon capture step wherein a source of carbon dioxide is contacted with an amine solution in a closed-top scrubber, to obtain an ammonium bicarbonate solution;
   (b) inducing crystallization in the ammonium bicarbonate solution to obtain a concentrated ammonium bicarbonate solution;
   (c) subjecting the concentrated ammonium bicarbonate solution to a hydrogenation step to obtain an ammonium formate; and
   (d) heating the ammonium formate to a temperature in the range of 50 - 150 °C, to obtain a gaseous product containing the amine and a liquid product stream containing formic acid,
   wherein the source of carbon dioxide has a carbon dioxide content of at least 95 vol% and the amine used in step (a) and reformed in step (d) has a partial vapour pressure above a 40 wt% solution of the amine in water at 20 °C of at least 40 kPa.
2. The process according to embodiment 1, wherein the amine is ammonia, monomethylamine, dimethylamine or trimethylamine, preferably trimethylamine.
3. The process according to embodiment 1 or 2, wherein the amine solution comprises 20 - 55 wt%, preferably 20 - 40 wt% of the amine, preferably wherein the remainder of the solution consists of water and a water-miscible organic solvent in a weight ratio water to organic solvent in the range of 5/95 - 95/15.
4. The process according to any one of the preceding embodiments, wherein the amine scrubber does not contain a gas outlet located at the top part of the scrubber that would allow the release of a gaseous effluent from the scrubber, preferably the scrubbed is gas-tight, such that gases can only exit the scrubber in solution via a liquid outlet at the bottom part of the scrubber.
5. The process according to any one of the preceding embodiments, wherein the crystallization of step (b) is induced by lowering the temperature of the ammonium bicarbonate solution to a temperature in the range of 0 - 15 °C, preferably in the range of 3 - 5 °C, and preferably wherein the slurry is heated to a temperature in the range of 50 - 100 °C, more preferably in the range of 70 - 90 °C before it is subjected to the hydrogenation of step (c).
6. The process according to any one of the preceding embodiments, wherein the hydrogen gas required in step (c) originates from electrolysis of water.
7. The process according to any one of the preceding embodiments, wherein the ammonium formate originating from step (c) is subjected to step (e) to remove residual hydrogen gas, preferably in a flash drum, before it is subjected to heating of step (d), preferably wherein the hydrogen gas is recycled to the hydrogenation of step (c).
8. The process according to any one of the preceding embodiments, wherein the ammonium bicarbonate concentration of the concentrated ammonium bicarbonate solution is in the range of 20 - 40 wt% when it is subjected to step (c).
9. The process according to any one of the preceding embodiments, wherein the heating of step (d) is performed in a stripper column, wherein the gaseous product is obtained as top gas and the liquid product stream as bottom effluent.
10. The process according to any one of the preceding embodiments, further comprising a step (g) wherein the formic acid is subjected to a hydrogenation step to obtain formaldehyde.
11. The process according to any one of the preceding embodiments, wherein the gaseous product obtained in step (d) is subjected to condensation in step (f), wherein residual formic acid and water are removed from the amine, preferably wherein the amine is recycled to step (a) and/or the mixture of formic acid and water is recycled back to step (d).
12. The process according to any one of the preceding embodiments, wherein the amine obtained in step (d), optionally after condensation step (f), is recycled back to step (a).
13. The method according to any one of the preceding embodiments, wherein the source of carbon dioxide has a carbon dioxide content of at least 99 vol%.
14. A modular system for performing the process according to any one of embodiments 1 - 13, comprising:
   (a) a carbon capture module comprising a closed-top amine scrubber having a first inlet for receiving an amine solution, a second inlet for receiving a source of carbon dioxide, and an outlet in the bottom part of the scrubber for discharging an ammonium bicarbonate solution;
   (b) a crystallization module comprising means to crystallize ammonium bicarbonate, preferably wherein the means include a cooler configured to cool the ammonium bicarbonate solution to a temperature in the range of 0 - 15 °C, and an outlet for discharging an ammonium bicarbonate slurry;
   (c) a hydrogenation reactor, comprising an inlet for receiving the slurry, an inlet for receiving hydrogen gas, and an outlet to discharge an ammonium formate solution; and
   (d) a stripper or distillation column, comprising an inlet for receiving the ammonium formate solution, an gas outlet at the top part of the stripper for discharging a gaseous product containing the amine and a liquid outlet at the bottom part of the stripper for discharging a liquid product stream containing formic acid.
15. The modular system according to embodiment 14, further comprising one or more modules selected from:
   (e) a vapour-liquid separator for separating hydrogen gas from the ammonium formate solution from module (c), to obtain an ammonium formate solution depleted in hydrogen gas to be subjected to module (d) and hydrogen gas;
   (f) a condenser for subjecting the gaseous product from module (d) to condensation, to obtain a liquid stream containing formic acid and water, and a gaseous stream containing the amine; and
   (g) a second hydrogenation reactor, comprising an inlet for receiving the liquid stream containing formic acid from module (d) or (f), an inlet for receiving hydrogen gas, and an outlet for discharging formaldehyde.

### Detailed description

First and foremost, the invention concerns a highly efficient and durable process for the production of formic acid from carbon dioxide. In a second aspect, the invention concerns a system for performing the process according to the invention. Here below, the process and system according to the invention are separately described. Nonetheless, the skilled person will appreciate that everything said for the process applies also the system and everything said for the system applies also for the process.

### Process

In a first aspect, the invention concerns a process for the formation of formic acid from a source of carbon dioxide.

The process according to the invention comprises:
(a) a carbon capture step wherein a source of carbon dioxide is contacted with an amine solution in a closed-top scrubber, to obtain an ammonium bicarbonate solution;
(b) inducing crystallization in the ammonium bicarbonate solution to obtain a concentrated ammonium bicarbonate solution;
(c) subjecting the concentrated ammonium bicarbonate solution to a hydrogenation step to obtain an ammonium formate; and
(d) heating the ammonium formate to a temperature in the range of 50 - 150 °C, to obtain a gaseous product containing the amine and a liquid product stream containing formic acid.

Herein, the source of carbon dioxide has a carbon dioxide content of at least 95 vol% and the amine used in step (a) and reformed in step (d) has a partial vapour pressure above a 40 wt% solution of the amine in water at 20 °C of at least 40 kPa. Herein, "ammonium" refers to any quaternary amine, which may be formed from ammonia or a substituted amine.

In step (a), the capture of carbon dioxide in an amine solution takes place. Such carbon capture is known in the art and typically takes place in an amine scrubber, also referred to as an absorber. The scrubber is fed with a source of carbon dioxide and an amine solution. In order to optimize contact between the amine and the carbon dioxide, the carbon dioxide is typically fed via a gas inlet at the bottom of the scrubber and the amine solution is fed via a liquid inlet at the top of the scrubber. During step (a), the carbon dioxide that is fed to the scrubber reacts with the amine present within the scrubber and forms an ammonium bicarbonate salt, possibly together with other carbon oxide species such as carbonate and carbamate anions. The thus formed ammonium bicarbonate solution is discharged from the scrubber from a liquid outlet at the bottom of the scrubber.

The present invention makes it possible to use volatile amines, because the scrubber is a closed top scrubber. A closed-top scrubber refers to a scrubber lacking a gaseous outlet at the top of the scrubber (such as a top vent), or wherein this outlet is kept closed during the process in operation. Preferably, a closed-top scrubber lacking a gaseous outlet at the top. Herein, "closed" refers to closed for the release or the discharge of gases. Preferably, the amine scrubber does not contain a gas outlet located at the top part of the scrubber that would allow the release of a gaseous effluent from the scrubber, more preferably the scrubbed is gas-tight, such that gases can only exit the scrubber in solution via a liquid outlet at the bottom part of the scrubber. Crucial in the context of the present process is that no gases are released at the top of scrubber during step (a). Any gas present in the scrubber may be released in solution via the liquid outlet at the bottom of the scrubber.

Suitable amines for carbon capture processes are knows to the skilled person. The amine that is used in step (a) for capturing the carbon dioxide should have a certain volatility for the process to operate smoothly. Preferably, the volatility of the amine is preferably expressed as the vapour pressure at 20 °C, wherein a vapour pressure of at least 10 kPa is required. Preferably, the vapour pressure is at least 50 kPa or even at least 150 kPa, such as is in the range of 10 - 1000 kPa, preferably 50 - 500 kPa or even 150 - 250 kPa. Herein, the vapour pressure of the amine in the amine solution that is used in step (a) is relevant. Thus, in an alternative embodiment, the volatility is expressed with respect to the volatility of a 20 wt% ammonia solution in water at 20 °C. Herein the volatility of the aqueous amine solution is expressed as the partial vapour pressure of the amine in the gas mixture above the liquid. For a 20 wt% aqueous ammonia solution at 20 °C, this is about 29 kPa (Perman, Jour. Chem. Soc. 1903, 83, 1168). Preferably, the volatility of the amine in the amine solution is at least 80 %, preferably at least 95 %, most preferably at least 100 % of the volatility of the 20 wt% amine solution defined above. In other words, in a preferred embodiment, the partial vapour pressure of the amine above a 20 wt% solution of the amine in water at 20 °C is at least 23 kPa, preferably at least 27 kPa, more preferably at least 29 kPa or even at least 40 kPa. Alternatively, the partial vapour pressure of the amine above a 40 wt% solution of the amine in water at 20 °C is at least 40 kPa, preferably at least 50 kPa, more preferably at least 60 kPa or even at least 90 kPa. In one embodiment, the amine is selected from ammonia, monomethylamine (MMA), dimethylamine (DMA) and trimethylamine (TMA), which are highly suitable in view of their volatility. A 40 wt% trimethylamine solution in water has a partial pressure of 67 kPa, and a 40 wt% of ammonia in water has a partial pressure of 107 kPa, which makes both perfectly suitable amines in the context of the present invention. Even though it is not considered a commericially sound process, carbon capture using trimethylamine is known form e.g. Boo et al., J. Membrane Sci., 2015, 473, 302-309.

Furthermore, the amine should be capable of forming a bicarbonate salt when contacted with carbon dioxide. Although the process according to the invention is capable of removing ammonium carbonate and ammonium carbamate, if formed during step (a), by virtue of step (b) as described below, only the bicarbonates are converted into formic acid. No conversion of the ammonium carbonates and ammonium carbamates was observed. Thus, it is preferred that the amine forms ammonium bicarbonate is predominant product in step (a). Especially suitable amines in the context of the present invention include tertiary amines and ammonia, in particular ammonia and trimethylamine. In view of its high volatility, the use of trimethylamine is most preferred. The skilled person may find further guidance in selecting suitable amines in Mani et al. Green Chem. 2006, 8, 995-1000.

Although it has been suggested to use such volatile amines, there is a prejudice in the art to do so. In the art, there is consensus that highly volatile amines, such as MMA, DMA and TMA, are undesirable for carbon capture application, because the amines easily escape from the absorber wherein the carbon capture process is performed. It requires significant effort and energy to keep the amine in liquid form and contained in the absorber, e.g. using condensers. The use of such measures is avoided in the process according to the present invention, as instead a closed-top scrubber is used. Additionally, the use of bicarbonate forming amines is normally considered undesirable, as the bicarbonate salt has a high tendency to precipitate or crystallize, which would normally hinder downstream processing. In the process according to the present invention, the crystallization capacity of ammonium bicarbonate salts is advantageously used to increase the bicarbonate content of the effluent of the amine scrubber.

Additionally, since the amine used in step (a) is volatile, it is preferred that the source of carbon dioxide does not contain substantial amounts of volatile species other than carbon dioxide. The carbon dioxide will react with the amine and discharged via the liquid outlet. Other gaseous species typically remain gaseous and unreacted and may accumulate in the closed-top scrubber. In order to avoid such accumulation, it is preferred that the source of carbon dioxide has a carbon dioxide content of at least 95 vol%, preferably at least 99 vol%. Traces of inert gaseous components such as nitrogen can be accommodated in the present process, and will be discharged in solution via the liquid outlet.

In light of the overall aim to provide a durable process for the production of formic acid, it is preferred that the carbon dioxide originates from a flue gas or combustion gas, which may otherwise be emitted into the environment. It is thus preferred that the carbon dioxide as isolated from the other gaseous components in the flue gas prior to being subjected to step (a). Such isolation of carbon dioxide is known in the art. Typically, a regular carbon capture process can be used to obtain such a high-purity carbon dioxide source, or a high-purity carbon dioxide stream of any industrial process can be used.

Although any concentration of the amine in the amine solution, typically the aqueous amine solution, is suitable in the context of the present process, it is preferred that the concentration is as high as possible. Higher concentrations lead to increased ammonium bicarbonate concentrations, which eventually gives higher formic acid yields. Thus, the amine concentration may be as high possible, as long as the solution remains in liquid form in the absorber. The amine solution that is fed to the scrubber typically comprises 10 - 60 wt%, preferably 15 - 50 wt%, more preferably 20 - 45 wt%, most preferably 20 - 40 wt% of the amine. Such amine concentrations are increased with respect to conventional amine concentrations used in carbon capture. Such an increased amine concentration ensures that the ammonium bicarbonate solution obtained in step (a) has a similar high concentration, and ammonium bicarbonate is easily precipitated in step (b). Additionally, the amount of other solvents, typically including at least water, is kept at a minimum, which makes downstream processing in terms of stream volumes and water removal more efficient.

The amine solution further comprises water. In one embodiment, the remainder of the amine solution is water. In an alternative and preferred embodiment, the amine composition comprises water and a lower-boiling water-miscible organic solvent. Lower boiling refers to the boiling point being lower than that of water, i.e. below 100 °C. Preferably, the boiling point of the water-miscible organic solvent is in the range of 25 - 90 °C, more preferably in the range of 40 - 75 °C (at ambient pressure). It is known that replacing part of the water in the amine solution does not hamper the carbon dioxide capture process. The skilled person finds guidance in Heldebrant et al. in Chem. Rev. 2017, 117, 9594-9624 for suitable solvents. Especially suitable organic solvents include methanol, ethanol, Preferably, the solvent system, in which the amine is dissolved contains water and the water-miscible organic solvent in a weight ratio water to organic solvent in the range of 5/95 - 95/5, preferably in the range of 10/90 - 60/40, most preferably 10/90 to 30/70. The use of a lower-boiling water-miscible organic solvent to replace part of the water in the amine solution was found to advantageously affect the thermal decomposition step (d), as described below.

In step (d), the amine that is used in step (a) is retrieved again. The process according to the invention may contain a recycle, wherein the amine that is formed in step (d) is recycled to step (a) as part of the amine solution that is fed to the scrubber.

The ammonium bicarbonate solution that is discharged from the amine scrubber is subjected to a crystallization step (b). Step (b) affords a slurry containing ammonium bicarbonate crystals. Crystallization may be induced by any means known in the art. In a preferred embodiment, crystallization is induced by lowering the temperature of the ammonium bicarbonate solution to a temperature in the range of 0 - 15 °C, preferably in the range of 3 - 5 °C. The cooling of the slurry may be performed in a crystallization vessel as known in the art.

The crystallization step (b) induces crystallization of ammonium bicarbonate salts. If, during step (a), some ammonium carbonate and/or ammonium carbamate is produced, these salts will remain largely in solution, as there solubility in water is greater than for the corresponding ammonium bicarbonates. Any formed ammonium carbonate and/or ammonium carbamate is thus largely removed together with the liquid phase during step (b). As such, crystallization step (b) ensures that the process can cope with ammines that form ammonium carbonate and/or ammonium carbamate, as those will be removed prior to subjection the ammonium bicarbonate solution to the hydrogenation of step (c). Nonetheless, since only ammonium bicarbonate will be converted into formic acid, it is preferred that the amount of carbonate and carbamate salts formed during step (a) is as low as possible. The skilled person knows to select an amine for the preferred or even selective formation of ammonium bicarbonates. Especially tertiary amines and ammonia are suitable in that respect.

In step (b), solvent is removed from the crystals to further increase the concentration. Even though the carbon capture of step (a) may operate at higher concentrations than conventional capture processes, for efficient hydrogenation it is preferred that the concentration is even further increased. Preferably, step (b) affords a slurry containing crystals of ammonium bicarbonate wherein the overall ammonium bicarbonate concentration is in the range of 20 - 40 wt%. At such concentrations, ammonium formate is efficiently formed in the hydrogenation step (c).

After step (b), the slurry containing the bicarbonate is preferably heated to a temperature in the range of 50 - 100 °C, more preferably in the range of 70 - 90 °C. Such an increase in temperature ensures that the crystals are completely dissolved and that there is no longer a slurry, but instead a solution. The ammonium bicarbonate solution is then subjected to the hydrogenation of step (c). Although a slurry containing crystals could be subjected as such to the hydrogenation step, processing of the liquid streams is facilitated when the crystals are dissolved and a solution is subjected to the hydrogenation reactor. Additionally, hydrogenation is performed at elevated temperatures, such that heating is required anyway. Heating the slurry prior to step (c) can thus be performed at no additional energy expenditure.

In step (c), the ammonium bicarbonate is subjected to a hydrogenation step, wherein it is contacted with a hydrogenation catalyst in the presence of hydrogen gas and converted into ammonium formate. Such conversion of ammonium bicarbonate to ammonium formate is known in the art and can be performed by the skilled person in any suitable way. The skilled person may find further guidance on performing step (c) in Su et al. ChemSusChem, 2015, 8(5), 813-816. Herein, conversions of up to 95.6 % have been reported using a Pd/Ac catalyst. Alternative catalysts, such as Pd/C, may also be used and are apparent to the skilled person. Step (c) is typically performed in a hydrogenation reactor employing conditions effective for converting ammonium bicarbonate into ammonium formate.

In the context of the present invention, any source of hydrogen gas can be used. In light of the overall aim of providing a durable solution for the production of formic acid, it is preferred that the hydrogen gas originates from a renewable source. In a preferred embodiment, the hydrogen gas for step (c) originates from electrolysis of water.

The product of step (c) may contain unreacted hydrogen gas. In one embodiment of the process according to the invention, this residual hydrogen gas as separated from the ammonium formate in step (e). Thus, it is preferred that the ammonium formate originating from step (c) is subjected to step (e) to remove residual hydrogen gas, preferably using a vapour-liquid separator, before it is subjected to heating of step (d), preferably wherein the hydrogen gas is recycled to the hydrogenation of step (c). Such a hydrogen separation step may be performed in a flash drum. Thus, in a preferred embodiment, the hydrogen gas subjected to step (c) is a mixture of hydrogen gas originating from the electrolysis of water and a recycle from the downstream hydrogen removal step.

In step (d), the ammonium formate is further converted in formic acid via thermal decomposition. The inventors have developed a highly efficient method to do so, without use of polluting concentrated acids such as sulfuric acid, and without the formation of substantial waste streams. In order to efficiently convert ammonium formate into formic acid, it is heated to a temperature effective to split the ammonium formate into formic acid and an amine. Herein, the amine is the same amine as used in step (a), which is retrieved in step (d). The ammonium formate is typically heated to a temperature in the range of 50 - 250 °C, preferably in the range of 100 - 200 °C, most preferably 120 - 150 °C. Step (d) may also be referred to as the thermal decomposition of ammonium formate into formic acid and amine. Temperatures above 250 °C are especially undesirable, since that may lead to carbonisation and carbon black formation. The inventors have found that the process works efficiently at 130 °C, thus such high temperatures are not required. The pressure at which step (d) is performed is not crucial to the working of the process of the invention, and may for example be in the range of 0.5 - 10 bar, preferably 1 - 5 bar.

The heating typically occurs in a stripper column or a distillation column. Conveniently, the liquid ammonium formate solution is fed via a liquid inlet, which may be placed at any position in the column, such as at the top of the column or about halfway. Conveniently, the column is equipped with a reboiler at the bottom which heats the liquid within the column. The column, typically the reboiler, may contain a heat exchanger, wherein heat is transferred to the liquid in the column by means of a heating medium, such as thermal oil or a warm gas. In a preferred embodiment, steam is used to heat the liquid in the stripper or distillation column. Such columns are known in the art.

The liquid product stream, comprising formic acid and water, is discharged at the bottom of the column, while the gaseous product stream, comprising the amine, is collected at the top. Because of the use of a volatile amine in step (a), the amine is efficiently split from the formic acid in the stripper column and is a gaseous state such that it is easily separated from the formic acid product. Thus, even though the use of volatile amines in carbon capture is not considered a viable option in the art, the present inventors have used the volatility to their advantage by developing an efficient conversion of ammonium formate into formic acid. The inventors further found that the use of a lower-boiling water-miscible organic solvent in the amine solution employed in step (a) further improved the separation in the column. Since less water is present in the ammonium formate composition that is fed to step (d) contains less water, the liquid product stream collected at the bottom of the column is more concentrated in formic acid. In other words, a more concentrated formic acid stream is obtained as product, which improves the applicability of the formic acid. The lower-boiling water-miscible organic solvent is more volatile and is collected at the top of the column, together with the amine.

The gaseous effluent, typically obtained at the top of the stripper column, may contain traces of formate. Typically, the conditions that apply at the top of the column are such that formic acid and amine is revered back to ammonium formate. Advantageously, the gaseous effluent is subjected to a condensation step (f), wherein the residual ammonium formate and possibly water are removed from the amine and may be recycled back to the stripper column of step (d). Therein, it will split (again) into formic acid amine, after which the formic acid will end up in the liquid bottom effluent. As such, the gaseous amine stream is substantially pure and can be recycled to step (a). In case a lower-boiling water-miscible organic solvent is used as part of the amine solution, the gaseous effluent contains amine and the lower-boiling water-miscible organic solvent, which together can be recycled back to step (a), where they will form part of the amine solution.

The water of the amine solution that is fed to step (a) mostly ends up in the liquid product stream in step (d), which thus contains substantial amounts of water. Typically, a solution of 20 - 60 wt% formic acid in water is formed, preferably 25 - 40 wt% formic acid in water is formed. The mixture of formic acid and water is the main product of the process according to the invention. It may be used as deemed fit, e.g. as such or after separation. In one embodiment, the formic acid is further converted by hydrogenation into formaldehyde. In this embodiment, the process is not for the formation of formic acid, but for the production of formaldehyde, and the process further comprises a step (g) wherein the formic acid is subjected to a hydrogenation step to obtain formaldehyde. Such hydrogenation of formic acid to formaldehyde is known in the art.

The process according to the invention is typically performed in a closed system. The use of a closed amine scrubber for step (a) is already described above. Additionally, the entire system in which the process is performed is preferably gas-tight in order to avoid leakage of the volatile amine.

The process and system according to the invention allow for efficient heat integration. For example, heat that is released from the process during step (a) or (b) may be transferred to step (c) or the heating step in step (d), typically using a heat exchanger. Such use of heat exchanger to make efficient use of the heating and cooling steps within a process is well-known to the skilled person.

### System

The invention further concerns a modular system for performing the process according to the invention. The system according to the invention comprises:
(a) a carbon capture module comprising a closed-top amine scrubber having a first inlet for receiving an amine solution, a second inlet for receiving a source of carbon dioxide, and an outlet in the bottom part of the scrubber for discharging an ammonium bicarbonate solution;
(b) a crystallization module comprising means to crystallize ammonium bicarbonate, preferably wherein the means include a cooler configured to cool the ammonium bicarbonate solution to a temperature in the range of 0 - 15 °C, and an outlet for discharging an ammonium bicarbonate slurry;
(c) a hydrogenation reactor, comprising an inlet for receiving the slurry, an inlet for receiving hydrogen gas, and an outlet to discharge an ammonium formate solution; and
(d) a stripper or distillation column, comprising an inlet for receiving the ammonium formate solution, an gas outlet at the top part of the stripper for discharging a gaseous product containing the amine and a liquid outlet at the bottom part of the stripper for discharging a liquid product stream containing formic acid.

Preferably, the system further comprises one or more of the following modules:
(e) a vapour-liquid separator for separating hydrogen gas from the ammonium formate solution from module (c), to obtain an ammonium formate solution depleted in hydrogen gas to be subjected to module (d) and hydrogen gas;
(f) a condenser for subjecting the gaseous product from module (d) to condensation, to obtain a liquid stream containing formic acid and water, and a gaseous stream containing the amine;
(g) a second hydrogenation reactor, comprising an inlet for receiving the liquid stream containing formic acid from module (d) or (f), an inlet for receiving hydrogen gas, and an outlet for discharging formaldehyde.

Herein, module (a) is for performing step (a), module (b) is for performing step (b), module (c) is for performing step (c), module (d) is for performing step (d), module (e) is for performing step (e), module (f) is for performing step (f) and module (g) is for performing step (g). The modules of the system according to the invention are interconnected to allow fluid connectivity of the streams between the modules. Such fluid connectivities exist between the outlet of one module and the inlet of the subsequent module. In one embodiment, the system according to the invention comprises a vapour-liquid separator (e). In one embodiment, the system according to the invention comprises a condenser (f). In one embodiment, the system according to the invention comprises a second hydrogenation reactor (g). In a preferred embodiment, the system according to the invention comprises vapour-liquid separator (e) and condenser (f). In an especially preferred embodiment, the system according to the invention comprises vapour-liquid separator (e), condenser (f) and a second hydrogenation reactor (g).

Carbon capture modules are known in the art, and any suitable carbon capture module can be used as module (a). It comprises a closed-top amine scrubber having a first inlet for receiving an amine solution, a second inlet for receiving a source of carbon dioxide, and an outlet in the bottom part of the scrubber for discharging an ammonium bicarbonate solution. A closed-top scrubber refers to a scrubber lacking a gaseous outlet at the top of the scrubber (such as a top vent), or wherein this outlet is kept closed during the process in operation. Preferably, a closed-top scrubber lacking a gaseous outlet at the top. Herein, "closed" refers to closed for the release or the discharge of gases. Preferably, the amine scrubber does not contain a gas outlet located at the top part of the scrubber that would allow the release of a gaseous effluent from the scrubber, more preferably the scrubbed is gas-tight, such that gases can only exit the scrubber in solution via a liquid outlet at the bottom part of the scrubber.

Crystallization modules are known in the art, and any suitable crystallization modules can be used as module (b). It comprises means to crystallize ammonium bicarbonate, preferably wherein the means include a cooler configured to cool the ammonium bicarbonate solution to a temperature in the range of 0 - 15 °C, and an outlet for discharging an ammonium bicarbonate slurry. Typically, module (b) comprises a crystallization vessel, which may contain a stirrer, which facilitates the formation of ammonium bicarbonate crystals. In a preferred embodiment, module (b) comprises means to remove liquid from the formed crystals, or a dewatering device, such as a centrifuge, cyclone or filtration device. A dewatering device removes liquid from the crystals, affording a liquid stream and a slurry containing the ammonium bicarbonate crystals in reduced volume. Module (b) further preferably comprises means for heating the dewatered slurry, such that the crystals are dissolved again and a ammonium solution is discharged from module (b) and fed to module (c). However, the slurry containing ammonium bicarbonate crystals can also be subjected as such to the reactor of module (c).

Hydrogenation reactors are known in the art, and any suitable hydrogenation reactor can be used as module (c). It comprises an inlet for receiving the ammonium bicarbonate solution, an inlet for receiving hydrogen gas, and an outlet to discharge an ammonium formate solution. The hydrogenation reactor comprises a catalyst capable of performing a hydrogenation reaction to convert bicarbonate into formate.

Strippers and distillation columns are known in the art, and any suitable stripper or distillation column can be used as module (d). It comprises an inlet for receiving the ammonium formate solution, an gas outlet at the top part of the stripper for discharging a gaseous product containing the amine and a liquid outlet at the bottom part of the stripper for discharging a liquid product stream containing formic acid.

Vapour-liquid separators are known in the art, and any suitable vapour-liquid separator can be used as module (e). A vapour-liquid separator for separating hydrogen gas from the ammonium formate solution from module (c), to obtain an ammonium formate solution depleted in hydrogen gas to be subjected to module (d) and hydrogen gas. The vapour-liquid separator is preferably a flash drum.

Condensers are known in the art, and any suitable condenser can be used as module (f). The condenser (f) is capable of condensing the gaseous product from module (d) to obtain a liquid stream containing formic acid and water, and a gaseous stream containing the amine. The condenser comprises a liquid outlet for discharging the liquid stream containing formic acid and water, which is preferably connected via a recycle to the stripper or distillation column of module (d) to recycle the liquid stream to module (d). The condenser further comprises a gas outlet for discharging a gaseous stream containing the amine, which is preferably connected via a recycle to module (a), in particular the gaseous inlet thereof, to recycle the amine gas to module (a).

The system according to the invention may further comprise a second hydrogenation reactor (g), comprising an inlet for receiving the liquid stream containing formic acid from module (d) or (f), an inlet for receiving hydrogen gas, and an outlet for discharging formaldehyde. Hydrogenation reactors are known in the art, and any suitable hydrogenation reactor can be used as module (g).

### Examples

### Example 1

A solution of ammonium bicarbonate was prepared with 700 mg NH₄HCO₃ in 70 ml of milli-Q water. The solution was inserted in the tube of a hydrogenation reactor along with 290 mg of Pd/C catalyst. The reactor is enclosed and pressurized at 20 bar H₂. The hydrogenation of ammonium bicarbonate was investigated on the following conditions: Temperature = 80 °C; H₂ pressure = 20 bar, retention time = 2 h; stirring speed = 500 rpm. A yield of 87.5 % ammonium formate was obtained, as determined by HPLC analysis of the product mixture.

The product mixture of reactor outlet was subjected to thermal decomposition of the ammonium formate. The product mixture (pH of 7.1) was subjected to a distillation column wherein it was heated to 150 °C for one hour while being stirred at 400 rpm. A distillate (pH of 13.1) and residue (pH 4.1) were obtained. The compositions of the reactor outlet mixture, the distillate and the residue were determined by HPLC and FTIR analysis. The HPLC analyses showed that 57 % yield of formic acid was afforded in the residue. The distillate contained pure ammonia solution. The IR spectrum of the distillate, a peak at about 1000 cm⁻¹ indicates the presence of ammonia, which is absent in the IR spectrum of the residue, which in turn shows the characteristic peaks for formic acid at about 1200 cm⁻¹ and 1700 cm⁻¹, which are absent in the IR spectrum of the distillate. Also, the measured pH values indicate the formation of acid in the residue and base in the distillate. This demonstrates that formic acid and ammonia are formed in the distillation column, and formic acid ended up in the liquid residue, whereas ammonia ended up in the distillate.

### Example 2

An aqueous solution of trimethylammonium formate was prepared by mixing 22.3 g of TMA and 7.8 g of formic acid were diluted in 200ml of milli-Q water. The solution was then subjected to thermal decomposition in a distillation column. Distillation was performed at 150 °C for one hour while being stirred at 400 rpm. A distillate and residue were obtained. The compositions of the reactor outlet mixture, the distillate and the residue were determined by HPLC analysis, which showed that 49 % yield of formic acid was afforded in the residue. The distillate contained pure TMA solution. This demonstrates that formic acid and TMA are formed in the distillation column, and formic acid ended up in the liquid residue, whereas TMA ended up in the distillate.

## Claims

1. A process for producing formic acid, comprising:
(a) a carbon capture step wherein a source of carbon dioxide is contacted with an amine solution in a closed-top scrubber, to obtain an ammonium bicarbonate solution;
(b) inducing crystallization in the ammonium bicarbonate solution to obtain a concentrated ammonium bicarbonate solution;
(c) subjecting the concentrated ammonium bicarbonate solution to a hydrogenation step to obtain an ammonium formate; and
(d) heating the ammonium formate to a temperature in the range of 50 - 150 °C, to obtain a gaseous product containing the amine and a liquid product stream containing formic acid,
wherein the source of carbon dioxide has a carbon dioxide content of at least 95 vol% and the amine used in step (a) and reformed in step (d) has a partial vapour pressure above a 40 wt% solution of the amine in water at 20 °C of at least 40 kPa.

2. The process according to claim 1, wherein the amine is ammonia, monomethylamine, dimethylamine or trimethylamine, preferably trimethylamine.

3. The process according to claim 1 or 2, wherein the amine solution comprises 20 - 55 wt%, preferably 20 - 40 wt% of the amine, preferably wherein the remainder of the solution consists of water and a water-miscible organic solvent in a weight ratio water to organic solvent in the range of 5/95 - 95/5.

4. The process according to any one of the preceding claims, wherein the amine scrubber does not contain a gas outlet located at the top part of the scrubber that would allow the release of a gaseous effluent from the scrubber, preferably the scrubbed is gas-tight, such that gases can only exit the scrubber in solution via a liquid outlet at the bottom part of the scrubber.

5. The process according to any one of the preceding claims, wherein the crystallization of step (b) is induced by lowering the temperature of the ammonium bicarbonate solution to a temperature in the range of 0 - 15 °C, preferably in the range of 3 - 5 °C, and preferably wherein the slurry is heated to a temperature in the range of 50 - 100 °C, more preferably in the range of 70 - 90 °C before it is subjected to the hydrogenation of step (c).

6. The process according to any one of the preceding claims, wherein the hydrogen gas required in step (c) originates from electrolysis of water.

7. The process according to any one of the preceding claims, wherein the ammonium formate originating from step (c) is subjected to step (e) to remove residual hydrogen gas, preferably in a flash drum, before it is subjected to heating of step (d), preferably wherein the hydrogen gas is recycled to the hydrogenation of step (c).

8. The process according to any one of the preceding claims, wherein the ammonium bicarbonate concentration of the concentrated ammonium bicarbonate solution is in the range of 40 - 60 wt% when it is subjected to step (c).

9. The process according to any one of the preceding claims, wherein the heating of step (d) is performed in a stripper column, wherein the gaseous product is obtained as top gas and the liquid product stream as bottom effluent.

10. The process according to any one of the preceding claims, further comprising a step (g) wherein the formic acid is subjected to a hydrogenation step to obtain formaldehyde.

11. The process according to any one of the preceding claims, wherein the gaseous product obtained in step (d) is subjected to condensation in step (f), wherein residual formic acid and water are removed from the amine, preferably wherein the amine is recycled to step (a) and/or the mixture of formic acid and water is recycled back to step (d).

12. The process according to any one of the preceding claims, wherein the amine obtained in step (d), optionally after condensation step (f), is recycled back to step (a).

13. The method according to any one of the preceding claims, wherein the source of carbon dioxide has a carbon dioxide content of at least 99 vol%.

14. A modular system for performing the process according to any one of claims 1 - 13, comprising:
(a) a carbon capture module comprising a closed-top amine scrubber having a first inlet for receiving an amine solution, a second inlet for receiving a source of carbon dioxide, and an outlet in the bottom part of the scrubber for discharging an ammonium bicarbonate solution;
(b) a crystallization module comprising means to crystallize ammonium bicarbonate, preferably wherein the means include a cooler configured to cool the ammonium bicarbonate solution to a temperature in the range of 0 - 15 °C, and an outlet for discharging an ammonium bicarbonate slurry;
(c) a hydrogenation reactor, comprising an inlet for receiving the slurry, an inlet for receiving hydrogen gas, and an outlet to discharge an ammonium formate solution; and
(d) a stripper or distillation column, comprising an inlet for receiving the ammonium formate solution, an gas outlet at the top part of the stripper for discharging a gaseous product containing the amine and a liquid outlet at the bottom part of the stripper for discharging a liquid product stream containing formic acid.

15. The modular system according to claim 14, further comprising one or more modules selected from:
(e) a vapour-liquid separator for separating hydrogen gas from the ammonium formate solution from module (c), to obtain an ammonium formate solution depleted in hydrogen gas to be subjected to module (d) and hydrogen gas;
(f) a condenser for subjecting the gaseous product from module (d) to condensation, to obtain a liquid stream containing formic acid and water, and a gaseous stream containing the amine; and
(g) a second hydrogenation reactor, comprising an inlet for receiving the liquid stream containing formic acid from module (d) or (f), an inlet for receiving hydrogen gas, and an outlet for discharging formaldehyde.
